# EUROPEAN PATENT APPLICATION

(11) **EP 3 376 206 A1**
(43) Date of publication of application: **19.09.2018**
(21) Application number: 16864157.9
(22) Date of filing: 07.11.2016
(51) Int. Cl.: G01N 21/03, G01N 21/41, G01N 21/552, G01N 21/64, G01N 33/543, G01N 35/02, B01L 3/00

(54) **INSPECTION CHIP AND INSPECTION SYSTEM**

(30) Priority: 13.11.2015 JP 2015223370
(71) Applicant: Konica Minolta, Inc., Tokyo 100-7015 (JP)
(72) Inventor: MATSUO, Masataka, Tokyo 100-7015 (JP); UMETSU, Hiroshi, Tokyo 100-7015 (JP)
(74) Representative: Burton, Nick
(86) International application number: PCT/JP2016/082941
(87) International publication number: WO 2017/082196

(57) **Abstract**

A reduction in the inspection accuracy of an inspection system, caused by stray light, is prevented. Provided is an inspection chip for use in an inspection system that detects a subject captured by immunoreaction, the inspection chip including an optical measurement chip having, on a top surface, a reaction field formation surface on which a reaction field for allowing the immunoreaction to occur is formed, and including a prism having a reflected light emission surface that emits reflected light reflected by the reaction field formation surface; and a cartridge in which at least one well that stores a liquid including the subject is formed; wherein the cartridge is not located in a region where a first space and a second space are overlapped under the assumption that: the first space is sandwiched between a flat surface which includes an end side of the reflected light emission surface, the end side being closer to the reaction field, and which is perpendicular to the reflected light emission surface, and a flat surface which includes the reflected light emission surface; and the second space is sandwiched between one flat surface which is perpendicular to a top surface of the prism and a longer side of the top surface and which is in contact with one edge of the reaction field in the longitudinal direction of the prism, and other flat surface which is perpendicular to the top surface of the prism and a longer side of the top surface and which is in contact with other edge of the reaction field in the longitudinal direction of the prism.

## Description

### Technological Field

The present invention relates to an inspection chip for use in an inspection system that detects a subject captured by immunoreaction, and the inspection system.

### Background Art

A detection plate in which a sensing unit that detects a subject and a reagent holding unit with wells formed therein, for storing a reagent and a specimen, are integrated has been conventionally known (see, for example, Patent Literature 1) as a detection plate that detects a subject captured by immunoreaction in inspection or the like performed based on the principle of surface plasmon-field enhanced fluorescence spectroscopy (SPFS) or the like. The detection plate is used, with being secured to a predetermined stage provided on an inspection system, at the time of inspection.

### Citation List

### Patent Literature

Patent Literature 1: JP 2013-024607 A

### Summary

### Technical Problem

Meanwhile, a high-capacity reagent well (for example, a well that stores a waste liquid, a cleaning liquid, and the like.) in the reagent holding unit included in the detection plate is preferably disposed close to the sensing unit. The reason for this is because a reagent well with a large number of access times is generally high in capacity and thus such a well can be disposed close to the sensing unit, thereby resulting in decreases in the migration length of the stage and the migration length of a pipette in feeding of the reagent to the sensing unit, to result in a decrease in the measuring time in an inspection system.

If the high-capacity well is disposed close to the sensing unit, however, the high-capacity well may be illuminated with reflected light of excitation light, reflected by a metallic thin film in inspection, during emission thereof through a prism, thereby causing stray light to be generated.

When stray light is caused, a problem is that background light is increased during inspection, to thereby make the inspection state unstable, resulting in a reduction in the inspection accuracy of an inspection system.

An object of the present invention is to provide an inspection chip and an inspection system in which a reduction in inspection accuracy caused by stray light can be prevented.

### Solution to Problem

In order to achieve at least one object described above, the inspection chip of the present invention is an inspection chip for use in an inspection system that detects a subject captured by immunoreaction, the inspection chip including:
an optical measurement chip having, on a top surface, a reaction field formation surface on which a reaction field for allowing the immunoreaction to occur is formed, and including a prism having a reflected light emission surface that emits reflected light reflected by the reaction field formation surface; and
a cartridge in which at least one well that stores a liquid for use in the inspection system is formed;
wherein the cartridge is not located in a region where a first space and a second space are overlapped under the assumption that:
   the first space is sandwiched between a flat surface which includes an end side of the reflected light emission surface, the end side being closer to the reaction field, and which is perpendicular to the reflected light emission surface, and a flat surface which includes the reflected light emission surface; and
   the second space is sandwiched between one flat surface which is perpendicular to a top surface of the prism and a longer side of the top surface and which is in contact with one edge of the reaction field in the longitudinal direction of the prism, and other flat surface which is perpendicular to the top surface of the prism and a longer side of the top surface and which is in contact with other edge of the reaction field in the longitudinal direction of the prism.

The inspection chip of the present invention is also an inspection chip for use in an inspection system that detects a subject captured by immunoreaction, the inspection chip including:
an optical measurement chip having a reaction field formation surface on which a reaction field for allowing the immunoreaction to occur is formed, and emitting reflected light reflected by the reaction field formation surface; and
at least one well that stores a liquid to be used or used in the inspection system;
wherein the well is disposed at a position not overlapped with an optical path of the reflected light.

Furthermore, the inspection system of the present invention is an inspection system that detects a subject captured by immunoreaction, the inspection system including:
a light source that emits excitation light; and
an inspection chip including an optical measurement chip provided with a prism having, on a top surface, a reaction field formation surface on which a reaction field for allowing the immunoreaction to occur is formed, and a cartridge in which at least one well that stores a liquid to be used in the inspection system is formed;
wherein the cartridge is not located in an optical path region of reflected light generated due to reflection of the excitation light by the reaction field formation surface.

### Advantageous Effects of Invention

According to the present invention, a reduction in the inspection accuracy of an inspection system due to stray light can be prevented.

### Brief Description of the Drawings

[Fig. 1] Fig. 1 is a plan view illustrating the surface of an inspection chip according to an embodiment.
[Fig. 2] Fig. 2 is a perspective view of the inspection chip according to an embodiment which is viewed from the rear surface thereof.
[Fig. 3] Fig. 3 is a view illustrating the cross section of the inspection chip according to an embodiment.
[Fig. 4] Fig. 4 is a view illustrating the cross section of an optical measurement chip according to an embodiment.
[Fig. 5] Fig. 5 is a plan view of the optical measurement chip according to an embodiment in top-view.
[Fig. 6] Fig. 6 is a cross-sectional view of a well according to an embodiment.
[Fig. 7] Fig. 7 is a view illustrating a shape of the inspection chip according to an embodiment, the shape being obtained by partially depressing a wall surface of a large well so as not to be illuminated with reflected light.
[Fig. 8] Fig. 8 is a cross-sectional view illustrating a case where the bottom of a large well according to another embodiment is processed so that an optical path of reflected light is avoided.
[Fig. 9] Fig. 9 is a view illustrating the cross section of an optical measurement chip according to another embodiment.
[Fig. 10] Fig. 10 is a view illustrating a state where a pipette chip is accommodated in a space formed in the inspection chip according to an embodiment.
[Fig. 11] Fig. 11 is a view illustrating a state where the inspection chip according to an embodiment is secured to a stage on which an engraved portion is formed.

### Description of Embodiments

The inspection chip and the inspection system of the present invention encompass the following.

The inspection chip of the present invention is an inspection chip for use in an inspection system that detects a subject captured by immunoreaction, the inspection chip including:
an optical measurement chip having, on a top surface, a reaction field formation surface on which a reaction field for allowing the immunoreaction to occur is formed, and including a prism having a reflected light emission surface that emits reflected light reflected by the reaction field formation surface; and
a cartridge in which at least one well that stores a liquid for use in the inspection system is formed;
wherein the cartridge is not located in a region where a first space and a second space are overlapped under the assumption that:
   the first space is sandwiched between a flat surface which includes an end side of the reflected light emission surface, the end side being closer to the reaction field, and which is perpendicular to the reflected light emission surface, and a flat surface which includes the reflected light emission surface; and
   the second space is sandwiched between one flat surface which is perpendicular to a top surface of the prism and a longer side of the top surface and which is in contact with one edge of the reaction field in the longitudinal direction of the prism, and other flat surface which is perpendicular to the top surface of the prism and a longer side of the top surface and which is in contact with other edge of the reaction field in the longitudinal direction of the prism.

Thus, when reflected light of excitation light, reflected by a metallic thin film, is emitted from the prism in inspection, stray light due to illumination of a part of the cartridge, such as the well, with the reflected light can be prevented from being generated.

In addition, in the inspection chip of the present invention,
a plurality of wells with different capacities are formed in the cartridge, and
at least one well smaller in capacity than a well having the maximum capacity among the plurality of wells is formed between the well having the maximum capacity and the optical measurement chip.

Thus, the number of wells can be increased with the cartridge being kept small in size.

In addition, in the inspection chip of the present invention,
a well is located in the vicinity of the optical measurement chip and is partially overlapped with the second space, and
an angle between a bottom wall surface rising from the bottom surface of the well towards a peripheral wall surface of the well, the peripheral wall surface being closer to the optical measurement chip, and the perpendicular line of the bottom surface of the well is an angle equal to or less than an angle between the perpendicular line of the reaction field formation surface and the reflected light emission surface.

Thus, even when a high-capacity well is disposed close to the optical measurement chip, the high-capacity well can be allowed not to be illuminated with the reflected light.

In addition, in the inspection chip of the present invention,
a plurality of wells with different capacities are formed in the cartridge, and
any bottom surface center of other well formed in the cartridge, excluding a well having the maximum capacity among the plurality of wells, is not located in the second space.

Thus, even when there is a well whose bottom surface is deep, the possibility can be reduced where the lower portion of the well is illuminated with the reflected light.

In addition, in the inspection chip of the present invention,
a rib that allows the cartridge to ensure rigidity is formed, and the rib is not located in a region where the first space and the second space are overlapped.

In addition, in the inspection chip of the present invention,
a wall surface of the well having the maximum capacity, the wall surface being located at a position overlapped with the second space and being closer to the optical measurement chip, is formed so as to be depressed in a direction opposite to the direction in which the optical measurement chip is located.

Thus, a high-capacity well can be more certainly prevented from being illuminated with the reflected light.

The inspection chip of the present invention is also an inspection chip for use in an inspection system that detects a subject captured by immunoreaction, the inspection chip including:
an optical measurement chip having a reaction field formation surface on which a reaction field for allowing the immunoreaction to occur is formed, and emitting reflected light reflected by the reaction field formation surface; and
at least one well that stores a liquid to be used or used in the inspection system;
wherein the well is disposed at a position not overlapped with an optical path of the reflected light.

In addition, in the inspection chip of the present invention,
the liquid is either a specimen or a reagent.

In addition, in the inspection chip of the present invention,
detection in the inspection system is performed using any of SPFS, SPR and ATR principles.

Furthermore, the inspection system of the present invention is an inspection system that detects a subject captured by immunoreaction, the inspection system including:
a light source that emits excitation light; and
an inspection chip including an optical measurement chip provided with a prism having, on a top surface, a reaction field formation surface on which a reaction field for allowing the immunoreaction to occur is formed, and a cartridge in which at least one well that stores a liquid to be used in the inspection system is formed;
wherein the cartridge is not located in an optical path region of reflected light generated due to reflection of the excitation light by the reaction field formation surface.

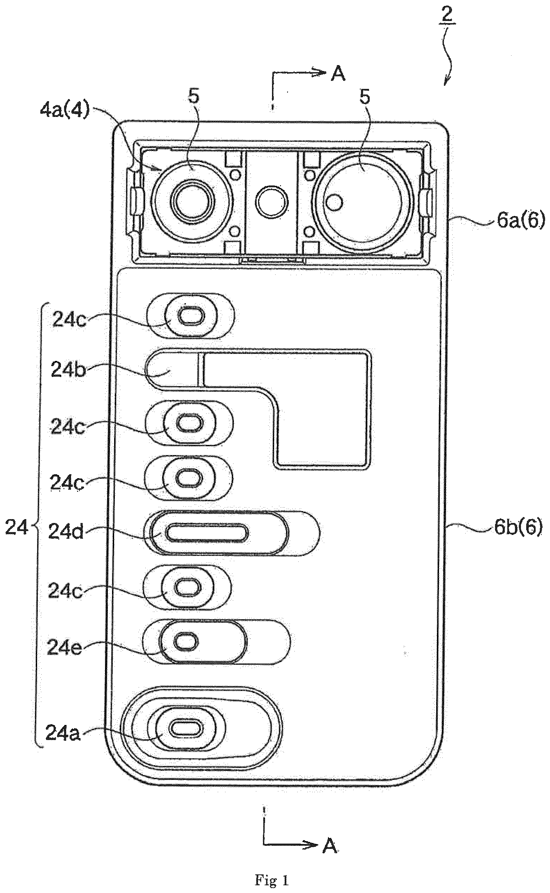

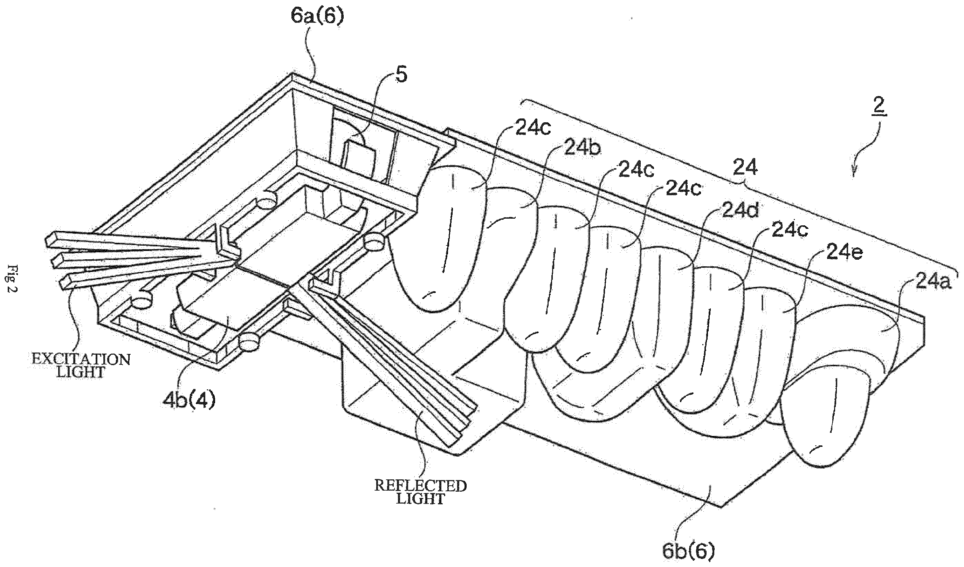

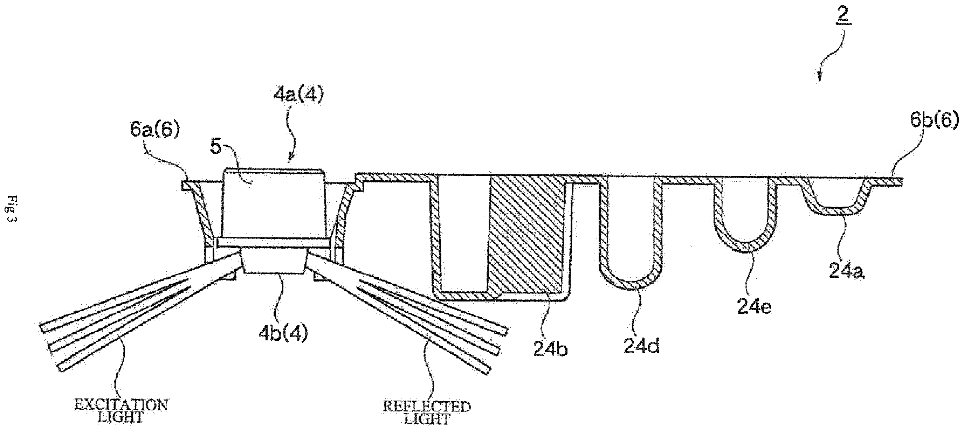

Hereinafter, an inspection chip according to an embodiment of the present invention will be described with reference to the drawings with an inspection chip to be used in an inspection system for SPFS measurement as an example. Fig. 1 is a plan view illustrating an inspection chip according to an embodiment, and Fig. 2 is a perspective view of the inspection chip which is viewed from the rear surface thereof. In addition, Fig. 3 is a view illustrating the A-A cross section in Fig. 1, of the inspection chip according to an embodiment.

As illustrated in Figs. 1 to 3, an inspection chip 2 includes a sensing member 4 having a substantially rectangular shape in planar view, and a cartridge 6 having a substantially rectangular shape in planar view, with the sensing member 4 disposed therein.

The sensing member 4 includes a flow channel chip 4a and an optical measurement chip 4b. The flow channel chip 4a is a chip disposed above the optical measurement chip 4b, and includes two cylindrical injection/ejection ports 5 through which liquids such as a specimen and a reagent are injected and ejected. Further, a flow channel (not illustrated) that guides a specimen and a reagent to a reaction field 12 is formed between the two injection/ejection ports 5.

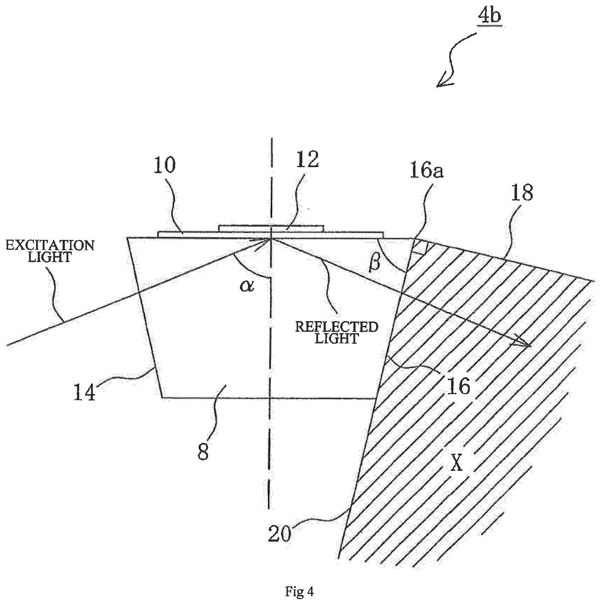

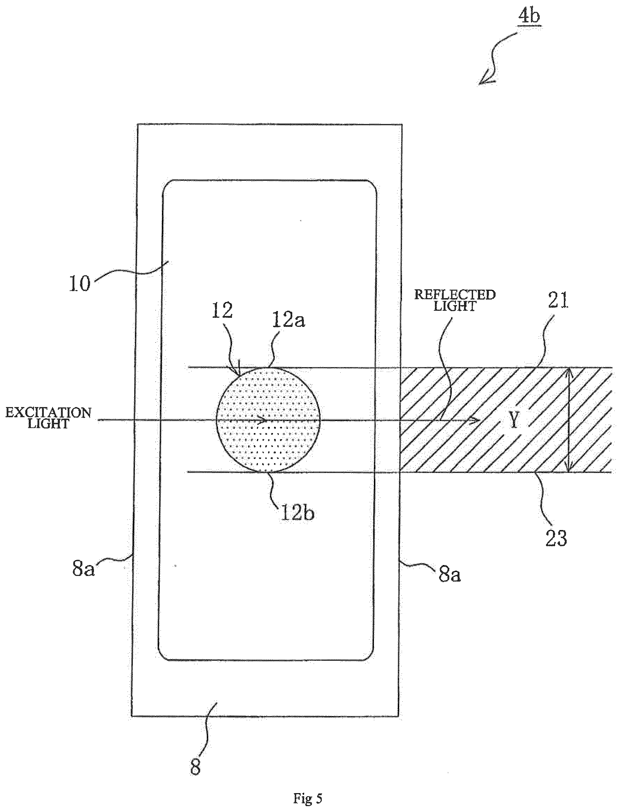

Fig. 4 is a view illustrating the cross section of the center portion in the longitudinal direction of the optical measurement chip 4b, and Fig. 5 is a plan view of the optical measurement chip 4b in top-view. As illustrated in Figs. 4 and 5, the optical measurement chip 4b includes a prism 8 as a dielectric member made of a hexahedron having a substantially trapezoidal cross section (truncated quadrangular pyramid shape), and a rectangular metallic thin film 10 (reaction field formation surface) disposed on the top surface of the prism 8. Herein, a reaction field 12 that allows a reaction product included in a specimen and/or a reagent to undergo immunoreaction is formed on the center portion of the metallic thin film 10.

Further, an excitation-light incident surface 14 upon which excitation light from a light source not illustrated is incident is formed on one side surface (left side surface in Fig. 4) of the prism 8, and a reflected light emission surface 16 to which reflected light reflected by the metallic thin film 10 is emitted is formed on other side surface (right side surface in Fig. 4) of the prism 8.

As illustrated in Fig. 4, it is here assumed with respect to the optical measurement chip 4b that there is a first space X sandwiched between a flat surface 18 which includes an end side 16a of the reflected light emission surface 16, the end side being closer to the reaction field 12, and which is perpendicular to the reflected light emission surface 16, and a flat surface 20 which includes the reflected light emission surface 16. Furthermore, as illustrated in Fig. 5, it is assumed that there is a second space Y sandwiched between one flat surface 21 which is perpendicular to the top surface of the prism 8 and a longer side 8a of the top surface and which is in contact with one edge 12a of the reaction field 12 in the longitudinal direction of the prism 8, and other flat surface 23 which is perpendicular to the top surface of the prism 8 and a longer side 8a of the top surface and which is in contact with other edge 12b of the reaction field 12 in the longitudinal direction of the prism 8. In this case, the cartridge 6 is formed so that the cartridge 6 is not located in a region where the first space X and the second space Y are overlapped. That is, the cartridge 6 is designed so as not to cause anywhere of a well formation portion 6b to be illuminated with reflected light emitted through the reflected light emission surface 16. Even when a rib is formed at any position of the cartridge 6 in order to enhance the strength of the cartridge 6 or suppress any strain to be generated on the cartridge by injection molding, the cartridge 6 is designed so as not to cause any rib to be formed in a region where the first space X and the second space Y are overlapped.

As illustrated in Figs. 1 to 3, the cartridge 6 is a substantially rectangular member in planar view, including a disposing portion 6a that disposes the sensing member 4, and a well formation portion 6b, and the cartridge 6 is formed by a resin member such as polystyrene or polypropylene. The disposing portion 6a includes an opening formed at one end in the longitudinal direction of the cartridge 6. In addition, a plurality of depressed wells 24 that store liquids such as a reagent and a specimen to be used in the inspection system are formed on the well formation portion 6b.

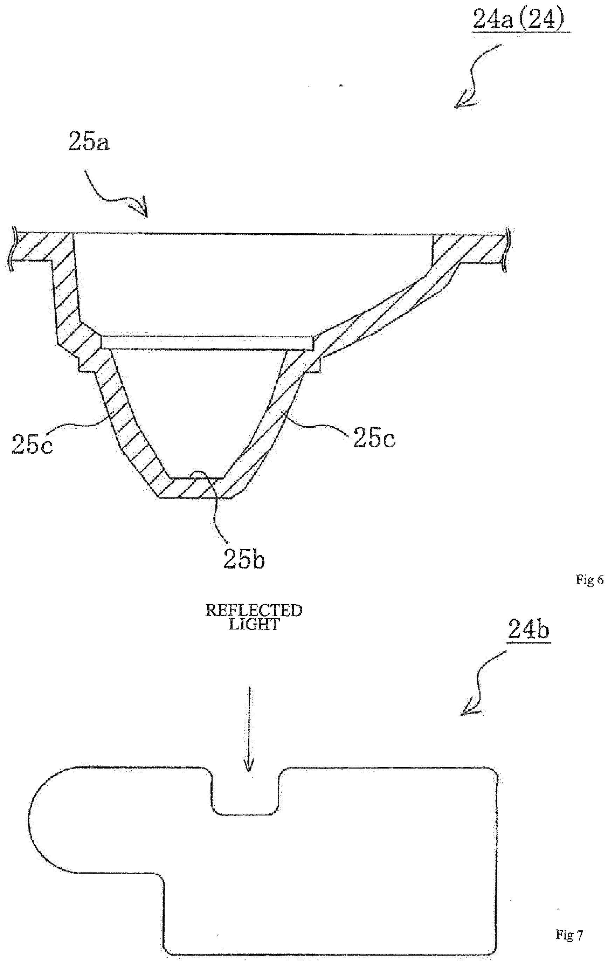

As illustrated in Fig. 6, each of the wells 24 (24a, 24b, 24c, 24d, 24e) has an opening portion 25a on the upper part thereof and has a bottom surface 25b smaller in the area than the opening portion 25a on the lower part thereof. In addition, the periphery of each of the wells 24 is surrounded by an inclined peripheral wall surface 25c. Therefore, when a liquid such as a specimen or a reagent is injected into any of the wells 24, the area of the liquid surface is expanded as the level of the liquid surface is raised. Fig. 6 illustrates the cross section of the well 24a located at a position farthest away from the optical measurement chip 4b in the cartridge 6.

A large well 24b having the maximum volume among the wells 24 has a substantially L-shaped opening portion 25a and a bottom surface 25b, and the wells 24 other than the large well 24b each have an opening portion 25a and a bottom surface 25b each having a substantially elliptical shape obtained by shaping each of both end portions in the longitudinal direction of a rectangle into an arc.

In addition, each of the wells 24 is disproportionately disposed in one region (left region in Fig. 1) in the width direction of the well formation portion 6b, and is formed so that the center of the bottom surface 25b of each of the wells 24 excluding the large well 24b is not located in the second space Y. Thus, if a deep well 24 whose bottom surface 25b is deep is included, the possibility can be reduced where the lower portion of the deep well 24 whose bottom surface 25b is deep is illuminated with the reflected light.

Furthermore, each of the wells 24 is formed so that the opening portion 25a extends in an elongated manner from a region (left region in Fig. 1), where the wells 24 are located, towards a region (right region in Fig. 1), where no wells 24 are located, in the well formation portion 6b of the cartridge 6. Thus, each of the wells 24, with the pitch therebetween being shortened in a lean way, can be disposed in the longitudinal direction of the well formation portion 6b and therefore the inspection chip 2 can be decreased in size.

The wells 24 include a plurality of wells 24 with different volume. A large well 24b having the maximum volume and with a large number of access times is here disposed in the vicinity of the optical measurement chip 4b in order to shorten the travel distance of a stage (not illustrated) provided in an inspection system (not illustrated) and shorten the measuring time in the inspection system. Furthermore, a small well 24c with small volume is disposed between the large well 24b and the optical measurement chip 4b (see Fig. 1). The small well 24c can be thus disposed between the large well 24b and the optical measurement chip 4b, thereby allowing the bottom surface 25b of the large well 24b not to be illuminated with the reflected light. In addition, the number of wells 24 can be increased with the size of the inspection chip 2 being kept small.

Next, a treatment where measurement based on the principle of SPFS is performed using the inspection chip 2 in an inspection system will be described with reference to a case where an analyte included in a specimen is detected as a reaction product. First, a specimen dispensed into any of the wells 24 formed in the cartridge 6 in the inspection chip 2 secured to the stage in the inspection system is, if necessary, diluted and mixed with a specimen dilution fluid.

Next, the specimen stored in any of the wells 24 is sucked once by a pipette chip not illustrated, and thereafter the specimen is injected into the flow channel chip 4a with which the upper surface of the optical measurement chip 4b is covered. The specimen injected into the flow channel chip 4a is guided by a flow channel not illustrated, to the metallic thin film 10, and captured by a ligand (antibody) where the analyte in the specimen is solid-phased in the reaction field 12, according to immunoreaction.

Next, a cleaning liquid is injected into the flow channel chip 4a to remove foreign substances from the specimen, thereafter a fluorescent substance is injected into the flow channel chip 4a, and the analyte in the specimen is labelled with the fluorescent substance. Next, a further cleaning liquid is injected into the flow channel chip 4a to remove the label nonspecifically adsorbing to the reaction field 12.

The specimen dilution fluid, the cleaning liquid and the fluorescent substance may be enclosed in any of the wells 24 formed in the cartridge 6. Further, the dilution of the specimen, to be if necessary performed, may also be performed in any of the wells 24 formed in the cartridge 6.

Next, excitation light is emitted towards the reaction field 12 from a light source disposed closer to the excitation-light incident surface 14 located at the lower portion of the prism 8 in the inspection system. The excitation light is incident through the excitation-light incident surface 14, and is reflected by the metallic thin film 10. As illustrated in Fig. 4, when the angle (namely, the incident angle of the excitation light) between the perpendicular line to the surface of the metallic thin film 10 and the excitation light is defined as "α" and the angle (hereinafter, the angle of the reflected light emission surface 16.) between the perpendicular line to the surface of the metallic thin film 10 and the reflected light emission surface 16 is defined as "β", the prism 8 is formed so that β ≥ α is satisfied.

When the excitation light is reflected by the metallic thin film 10, the excitation light with which the metallic thin film 10 is irradiated generates surface plasmon light (compression wave) on the metallic thin film 10, and the electric field enhancement effect of the surface plasmon light is achieved. Thus, the fluorescent substance bound to the analyte captured by the ligand in the reaction field 12 formed in the metallic thin film 10 is excited, and this fluorescence is observed, thereby resulting in detection of the analyte. For example, the intensity of the fluorescence is measured using a sensor that senses the amount of light, not illustrated, thereby resulting in detection of the analyte.

On the other hand, the reflected light reflected by the metallic thin film 10 is emitted through the reflected light emission surface 16. The inspection chip 2 is here designed so that the cartridge 6 is not located in the optical path region of the reflected light, and therefore the cartridge 6 is not illuminated with the reflected light emitted through the reflected light emission surface 16 and the reflected light does not serve as noise in fluorescence observation.

According to the inspection chip 2 according to this embodiment, there is not generated any stray light due to illumination of the large well 24b or the like with the reflected light emitted through the reflected light emission surface 16, and therefore a reduction in inspection accuracy due to stray light can be prevented.

In the above embodiment, as illustrated in Fig. 7, a wall surface of the large well 24b, being closer to the optical measurement chip 4b, may also be partially depressed in a direction opposite to the direction in which the optical measurement chip 4b is located, thereby allowing the shape of the large well 24b to be formed so that the lower portion of the large well 24b is not located in the second space Y. The large well 24b can be thus illuminated with no reflected light even if the large well 24b is close to the optical measurement chip 4b.

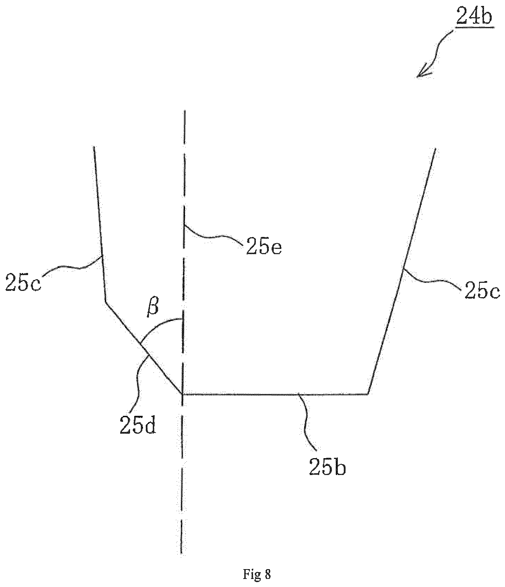

In addition, in the above embodiment, any well 24 (for example, large well 24b) located in the vicinity of the optical measurement chip 4b and partially overlapped with the second space Y may be subjected to predetermined processing. For example, as illustrated in Fig. 8, the angle between a bottom wall surface 25d rising up from the bottom surface 25b towards the peripheral wall surface 25c being closer to the optical measurement chip 4b and a perpendicular line 25e to the bottom surface 25b may be set to an angle equal to or less than the angle β of the reflected light emission surface 16. Also in this case, the large well 24b can be illuminated with no reflected light even if the large well 24b is close to the optical measurement chip 4b, as in the description with reference to Fig. 7.

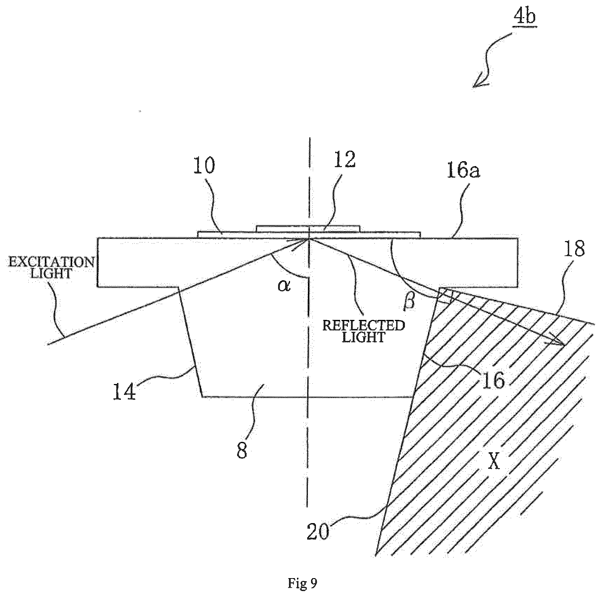

In addition, in the above embodiment, the prism 8 does not necessarily have the cross section shape illustrated in Fig. 4, and may have, for example, a shape where the top surface of the prism 8 is laterally put out, as illustrated in Fig. 9. In this case, the end side 16a of the reflected light emission surface 16, the end side being closer to the reaction field 12, is located more downward than the prism 8 shaped as illustrated in Fig. 4.

It has been described above in embodiments of the present invention that the cartridge 6 is not illuminated with the reflected light emitted through the reflected light emission surface 16 and the reflected light does not serve as noise in fluorescence observation. In an actual inspection system, the cartridge 6 is secured to a stage (not illustrated) and used. Therefore, even if the cartridge 6 is not illuminated with the reflected light, the stage can be illuminated with the reflected light and thus the reflected light can serve as noise in fluorescence observation.

Therefore, the stage to which the cartridge 6 is secured is desirably so as not to be illuminated with the reflected light emitted through the reflected light emission surface 16 in the vicinity of the reflected light emission surface 16, but to be illuminated therewith as far as possible. If the stage is illuminated with the reflected light in the vicinity of a reflecting surface, such an illumination location is close to the field of view of a light-receiving system and thus reflected light/scattering light at the location serves as noise in fluorescence observation.

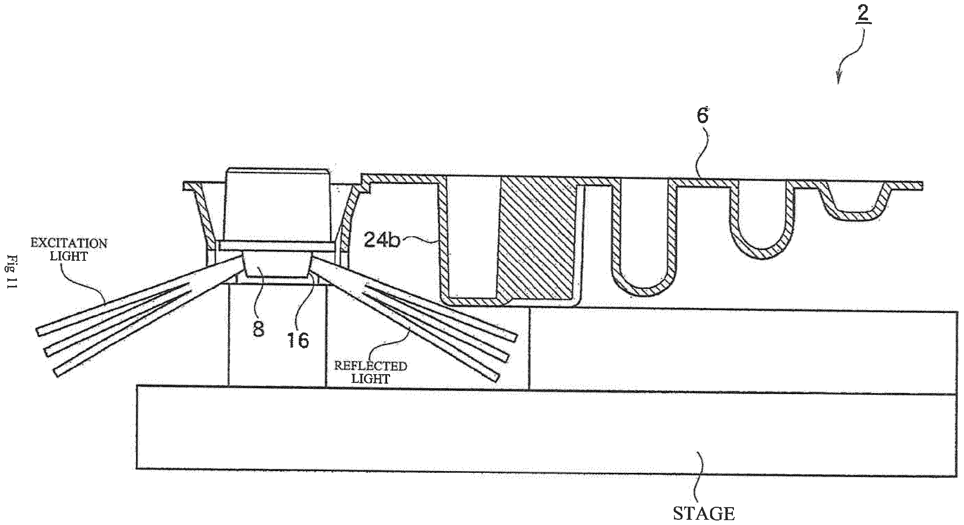

For example, when the cartridge 6 is secured onto the stage at the bottom thereof as in Fig. 11, the location of the stage to be illuminated with the reflected light is desirably far away by excavating the stage so that the stage is not immediately illuminated with the reflected light emitted through the reflected light emission surface 16. The target of the distance to the stage to be illuminated with the reflected light is desirably three times the size of the prism, and, for example, when the prism 8 is located 8 mm in the horizontal direction and 3 mm in the vertical direction far away, the distance from the reflecting surface to the stage is desirably 24 mm or more in the horizontal direction and 9 mm or more in the vertical direction.

A configuration as illustrated in Fig. 11, in which the position where the stage is to be illuminated with the reflected light is laid under the large well 24b of the cartridge 6, is desirably adopted because, even if the stage is illuminated with the reflected light, most of the reflected light/scattering light here is not directly returned in the prism direction, with hiding behind the cartridge 6, and does not serve as noise in fluorescence observation.

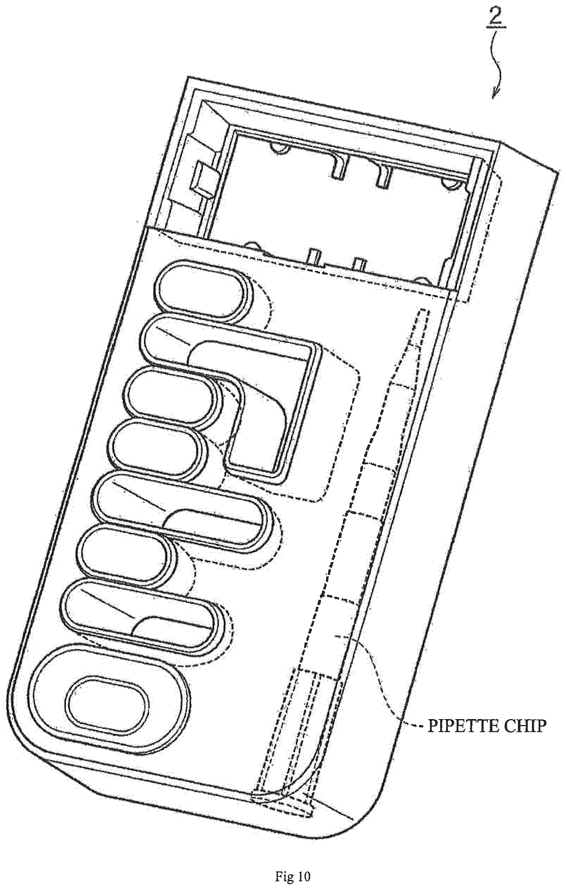

In the above embodiments, each of the wells 24 is disproportionately disposed in one width direction of the well formation portion 6b as illustrated in Fig. 1, and therefore a space having a predetermined width is generated on the rear surface of the well formation portion 6b. Therefore, a pipette chip may be accommodated in the space as illustrated in Fig. 10. The inspection chip 2 is sold with being put in a dedicated case, and if the inspection chip 2 by itself does not have any space that accommodates a pipette chip, any space that accommodates a pipette chip is needed to be provided in the case. The space that accommodates a pipette chip is here needed to be separately provided from a space that accommodates the inspection chip 2, and a problem is that the case is increased in size. On the contrary, as illustrated in Fig. 10, a space that accommodates a pipette chip can be provided in the inspection chip 2, thereby allowing the inspection chip 2 to be exported and sold with being accommodated in a small case not having any space that accommodates a pipette chip.

Although the description of the above embodiments is made with, as an example, an inspection chip to be used in an inspection system for SPFS measurement, the inspection system is not necessarily one for SPFS measurement, and for example, may be used as, for example, an inspection system for attenuated total reflectance (ATR) measurement and an inspection system for surface plasmon resonance (SPR) measurement.

In the above embodiments, although no critical description is made in Fig. 4, the excitation light is actually refracted on the excitation-light incident surface 14 and then is incident into the prism 8. The reflected light is also refracted on the reflected light emission surface 16 and emitted therethrough.

In the above embodiments, the opening portion 25a and the bottom surface 25b of each of the wells 24 may each have a circular or elliptical shape.

Although the description of the above embodiments is made with the metallic thin film 10 as the reaction field formation surface, the member to be used in the reaction field formation surface is not necessarily needed to be metallic.

In addition, in the above embodiments, the excitation-light incident surface 14 and the reflected light emission surface 16 are not necessarily a flat surface.

### Reference Signs List

2 inspection chip
4 sensing member
4a flow channel chip
4b optical measurement chip
6 cartridge
6b well formation portion
8 prism
10 metallic thin film
12 reaction field
12a one edge
12b other edge
14 excitation-light incident surface
16 reflected light emission surface
16a end side
18 flat surface including end side and being perpendicular to reflected light emission surface
20 flat surface including reflected light emission surface
21 one flat surface
23 other flat surface
24 well
24b large well
24c small well

## Claims

1. An inspection chip for use in an inspection system that detects a subject captured by immunoreaction, the inspection chip comprising:
an optical measurement chip having, on a top surface, a reaction field formation surface on which a reaction field for allowing the immunoreaction to occur is formed, and including a prism having a reflected light emission surface that emits reflected light reflected by the reaction field formation surface; and
a cartridge in which at least one well that stores a liquid for use in the inspection system is formed;
wherein the cartridge is not located in a region where a first space and a second space are overlapped under the assumption that:
the first space is sandwiched between a flat surface which includes an end side of the reflected light emission surface, the end side being closer to the reaction field, and which is perpendicular to the reflected light emission surface, and a flat surface which includes the reflected light emission surface; and
the second space is sandwiched between one flat surface which is perpendicular to a top surface of the prism and a longer side of the top surface and which is in contact with one edge of the reaction field in the longitudinal direction of the prism, and other flat surface which is perpendicular to the top surface of the prism and a longer side of the top surface and which is in contact with other edge of the reaction field in the longitudinal direction of the prism.

2. The inspection chip according to claim 1, wherein
a plurality of wells with different capacities are formed in the cartridge; and
at least one well smaller in capacity than a well having the maximum capacity among the plurality of wells is formed between the well having the maximum capacity and the optical measurement chip.

3. The inspection chip according to claim 1 or 2, wherein
a well is located in the vicinity of the optical measurement chip and is partially overlapped with the second space, and
an angle between a bottom wall surface rising from a bottom surface of the well towards a peripheral wall surface of the well, the peripheral wall surface being closer to the optical measurement chip, and a perpendicular line of the bottom surface of the well is an angle equal to or less than an angle between a perpendicular line of the reaction field formation surface and the reflected light emission surface.

4. The inspection chip according to claim 1, wherein
a plurality of wells with different capacities are formed in the cartridge, and
any bottom surface center of other well formed in the cartridge, excluding a well having the maximum capacity among the plurality of wells, is not located in the second space.

5. The inspection chip according to any one of claims 1 to 4, wherein a rib that allows the cartridge to ensure rigidity is formed, and the rib is not located in a region where the first space and the second space are overlapped.

6. The inspection chip according to claim 2 or 4, wherein a wall surface of the well having the maximum capacity, the wall surface being located at a position overlapped with the second space and being closer to the optical measurement chip, is formed so as to be depressed in a direction opposite to a direction in which the optical measurement chip is located.

7. An inspection chip for use in an inspection system that detects a subject captured by immunoreaction, the inspection chip comprising:
an optical measurement chip having a reaction field formation surface on which a reaction field for allowing the immunoreaction to occur is formed, and emitting reflected light reflected by the reaction field formation surface; and
at least one well that stores a liquid to be used or used in the inspection system;
wherein the well is disposed at a position not overlapped with an optical path of the reflected light.

8. The inspection chip according to any one of claims 1 to 7, wherein the liquid is either a specimen or a reagent.

9. The inspection chip according to any one of claims 1 to 8, wherein detection in the inspection system is performed using any of SPFS, SPR and ATR principles.

10. An inspection system that detects a subject captured by immunoreaction, the inspection system comprising:
a light source that emits excitation light; and
an inspection chip including an optical measurement chip provided with a prism having, on a top surface, a reaction field formation surface on which a reaction field for allowing the immunoreaction to occur is formed, and a cartridge in which at least one well that stores a liquid to be used in the inspection system is formed;
wherein the cartridge is not located in an optical path region of reflected light generated due to reflection of the excitation light by the reaction field formation surface.
